# FASCICULE DE BREVET EUROPEEN

(11) **EP 3 092 014 B1**
(45) Date de publication et mention de la délivrance du brevet: **11.07.2018**
(21) Numéro de dépôt: 15702540.4
(22) Date de dépôt: 08.01.2015
(51) Int. Cl.: A61L 27/10, A61L 27/36

(54) **PROCÉDÉ DE FABRICATION DE DISPOSITIFS D'OSTÉOSYNTHÈSE, DISPOSITIFS D'OSTÉOSYNTHÈSE ET IMPLANTS EN MATÉRIAU HYBRIDE SEMI-SYNTHÉTIQUE OBTENU PAR MODIFICATION STRUCTURALE DES COMPOSANTS D'UN BIOMATÉRIAU NATUREL MARIN**
VERFAHREN ZUR HERSTELLUNG VON OSTEOSYNTHESEVORRICHTUNGEN SOWIE DURCH STRUKTURÄNDERUNG DER KOMPONENTEN EINES NATÜRLICHEN MEERESBIOMATERIALS AUS EINEM HALBSYNTHETISCHEN HYBRIDMATERIAL HERGESTELLTE OSTEOSYNTHESEVORRICHTUNGEN UND IMPLANTATE
METHOD FOR MANUFACTURING OSTEOSYNTHESIS DEVICES, OSTEOSYNTHESIS DEVICES AND IMPLANTS MADE OF SEMI-SYNTHETIC HYBRID MATERIAL OBTAINED BY STRUCTURAL MODIFICATION OF THE COMPONENTS OF A NATURAL MARINE BIOMATERIAL

(30) Priorité: 10.01.2014 FR 1450204
(43) Date de publication de la demande: 16.11.2016
(73) Titulaire: MBP (Mauritius) Ltd, Port Louis (MU)
(72) Inventeur: CAMPRASSE, Georges, F-72230 Arnage (FR); CAMPRASSE, Serge, F-72200 La Fleche (FR)
(74) Mandataire: Cabinet Plasseraud
(86) Numéro de dépôt international: PCT/FR2015/050037
(87) Numéro de publication internationale: WO 2015/104502

(56) Documents cités:
- FR-A1- 2 946 888

## Description

### Domaine de l'invention

La présente invention concerne un matériau hybride semi-synthétique obtenu par modification structurale des composants d'un biomatériau naturel marin, en particulier la couche aragonitique nacrée des valves de mollusques marins tels que *Pinctada Maxima, Pinctada Margaritifera, Tridacnae Maxima, Tridacnae Gigas* et autres Pinctadines. La présente invention concerne aussi la fabrication de dispositifs d'ostéosynthèse et d'implants à partir dudit matériau hybride semi-synthétique.

### Contexte de l'invention

Le procédé le plus couramment utilisé lors de fractures, en dehors de la réduction de celles-ci par manoeuvres externes, est l'ostéosynthèse, qui consiste à réaligner les segments fracturés par l'utilisation de plaques, de vis, de clous et de fixateurs externes, fabriqués en acier inox ou en alliages titane, cobalt, etc.., de manière à immobiliser les fragments fracturés afin de permettre la formation d'un cal de cicatrisation.

La présence du matériel orthopédique tout au long de la période de consolidation, engendre des conditions mécaniques et métaboliques nouvelles, du fait que, d'une part, le matériel d'ostéosynthèse n'a pas les mêmes propriétés mécaniques et physiques que l'os, notamment module de Young, résistance à la flexion, élasticité, dureté, densité, et que, d'autre part, il subit l'action corrosive de l'environnement salin des liquides circulants du corps humain, action qui libère des microparticules métalliques, des ions, ainsi que divers sels métalliques. Il s'ensuit l'apparition de phénomènes inflammatoires qui peuvent provoquer la formation d'une enveloppe fibreuse, et générer douleur, oedème, infection, fistule, abcès, résorption et séquestres osseux à la périphérie de l'implant, manifestations qui peuvent apparaître plusieurs mois après l'implantation du matériel. C'est ainsi qu'on a pu, chez certains patients, pour lesquels le matériel orthopédique était implanté seulement depuis 2 ans, observer sur des prélèvements tissulaires avoisinant le matériel d'ostéosynthèse, un taux relativement élevé d'inclusions métalliques, d'inflammation chronique, de réactions d'oxydo-réduction, de corrosions galvaniques, de fibrose, de métallose ou de nécrose tissulaire, ce qui s'explique par une dégradation des plaques et un relargage d'ions métalliques toxiques tels que le cobalt, l'aluminium, etc...

Compte tenu de la nature métallique du matériel d'ostéosynthèse, une infection du site opératoire peut difficilement être traitée par voie générale, le métal s'opposant de par sa nature imperméable à la diffusion de l'agent thérapeutique. On a par ailleurs pu démontrer que, lors des manoeuvres de cintrage des plaques pour les adapter à la topographie du relief osseux du site d'implantation, cela provoquait des fissures et des amorces de rupture majorées par l'action des fluides circulants et par les contraintes mécaniques, du fait de la différence des caractéristiques physico-chimiques de l'os et du métal. En outre, on observe quelquefois, plusieurs mois, voire plusieurs années après l'intervention, une migration des plaques due à la mobilisation des vis de fixation, du fait que les propriétés tribologiques du métal peuvent difficilement être modifiées compte tenu de sa nature. En effet, toute modification de l'état de surface du métal peut le rendre vulnérable à la corrosion et altérer ses propriétés mécaniques. Afin d'améliorer les propriétés tribologiques du matériel d'ostéosynthèse et des implants métalliques, ils ont été recouverts d'un revêtement d'hydroxyapatite par un procédé de frittage par torche à plasma, dans le but d'obtenir l'adhésion d'os métaplasique à l'interface, lors de la cicatrisation osseuse. Cependant, dans la majorité des cas, il se produit un détachement du revêtement, la formation d'un tissu fibreux, avec pour corollaire la mobilisation du matériel d'ostéosynthèse ou des implants.

A l'origine, le matériel d'ostéosynthèse était appelé à être déposé dès que la consolidation de la fracture avait été constatée cliniquement et radiologiquement. Toutefois, dans un nombre non négligeable de cas, le matériel d'ostéosynthèse est laissé en place, car une ré-intervention pour le déposer nécessiterait une nouvelle hospitalisation, une intervention quasi-identique à la première, avec les complications habituelles liées à toute intervention chirurgicale, a fortiori sur l'os. En effet, l'ablation du matériel d'ostéosynthèse laisse un os comportant des trous de forage, une corticale amincie du fait d'un défaut de vascularisation provoqué par la pression de la plaque. Il en résulte une fragilisation secondaire du site opératoire avec possibilité de fracture.

Certains cas nécessitent l'ablation impérative du matériel d'ostéosynthèse, pour les raisons suivantes : plainte du patient, douleurs localisées, proéminence du matériel, infections localisées, pseudarthrose, migration et fracture du matériel, fracture osseuse péri-implantaire, toxicité et allergie.

L'ablation est également quasi-obligatoire dans les fractures de la clavicule nécessitant une prise en charge chirurgicale, compte tenu de la situation sous-cutanée et de la présence d'éléments vasculo-nerveux, tels que le plexus brachial et l'artère sous-clavière, ainsi que le risque de pseudarthrose résultant de l'ischémie provoquée par la pression de la plaque sur un os plat.

L'ablation s'avère également obligatoire en chirurgie orthopédique pédiatrique, qui n'obéit pas aux mêmes règles que celles de l'adulte ; en particulier, lorsque la fracture intéresse la région épiphyso-diaphysaire d'un os long, celle-ci englobe chez l'enfant la métaphyse, siège du cartilage de conjugaison, responsable de la croissance de l'os. La persistance d'un matériel d'ostéosynthèse à ce niveau durant un laps de temps trop long compromettra la croissance du membre. C'est pour ces raisons que, chez l'enfant, on procède précocement à l'ablation du matériel orthopédique, de manière à éviter cet inconvénient. Ce faisant, on observe quelquefois des pseudarthroses et des fractures par fragilisation de l'os, sans oublier les complications liées à l'ablation du matériel.

On sait d'autre part que, lors du traitement d'une fracture par ostéosynthèse, le déroulement de la consolidation osseuse est modifié. En effet, lors de la pose des plaques et vis en métal, alliage ou tout autre matériau, on est obligé d'évacuer l'hématome fracturaire alors qu'il contient toutes les cellules ostéo-compétentes, ainsi que les molécules telles que les substances mitogènes, les facteurs de croissance comme TGFβ, protéines ubiquitaires régulatrices de la croissance, de même que les PDGF. Cet hématome contient également les facteurs ostéoinducteurs BMP, FGF, IGF, ainsi que les éléments importants que sont les péricytes qui, libérés à partir de la lame basale de l'endothélium des capillaires lésés lors de la fracture, entrent dans le processus de stimulation de l'angiogénèse, de la synthèse du collagène, des protéoglycanes, de l'ostéocalcine, et dans l'initiation de la phagocytose.

L'absence de tous ces facteurs va ralentir de façon considérable la production du cal osseux. De plus, compte tenu du fait que, lors de la consolidation d'une fracture ostéosynthésée, le remodelage doit se poursuivre durant 18 mois minimum, la dépose du matériel orthopédique avant cette date pour quelque raison que ce soit, augmente les risques de complications telles que fractures, pseudarthrose ou infection.

En conclusion, l'ablation du matériel d'ostéosynthèse, nécessitant une ré-intervention sur un site ayant déjà fait l'objet d'un traumatisme, faisant suite à une première intervention agressive pour l'os et les tissus environnants, engendre des remaniements cicatriciels qui perturbent la restitution ad integrum de l'anatomie initiale et empêchent le repérage des structures anatomiques. Tout ceci va à l'encontre de la définition de l'ostéosynthèse, manoeuvre qui consiste à la consolidation de l'os fracturé en position anatomique sans risquer la faillite de l'implant, tout ceci nonobstant l'impact socio-économique qui en résulte.

Il existe donc un réel besoin d'avoir du matériel d'ostéosynthèse s'ajustant au mieux au site fracturaire, pouvant y être maintenu de manière pérenne et proposant une solution alternative aux problèmes liés à la présence et/ou à l'ablation du matériel d'ostéosynthèse métallique existant, tels que relargage toxique, métallose des tissus périprothétiques, effets systémiques et fragilisation de l'os.

Les inventeurs ont mis en évidence qu'il est possible d'obtenir un matériau satisfaisant à ces exigences, à partir d'un biomatériau hybride naturel qui est la couche aragonitique nacrée de mollusques bivalves choisis dans le groupe comprenant Pinctada Maxima, Pinctada Margaritifera, Tridacnae Maxima, Tridacnae Gigas et autres Pinctadines. Ils ont également prouvé que ce biomatériau peut être utilisé pour la fabrication de dispositifs d'ostéosynthèse et d'implants, tels que : plaques et vis d'ostéosynthèse, coins d'ostéotomie, diabolos, cales et cages inter-somatiques, clous centromédullaires, têtes humérales et fémorales, cavités glénoïdes, plateaux tibiaux, condyles fémoraux, corps vertébraux, hémi-maxillaires, os de la chaîne des osselets, ancres chirurgicales pour la réinsertion ligamentaire et tendineuse, gouttières gainantes pour la réduction ostéosynthésée de fractures comminutives à petits fragments, vis de contention membranaire et implants dentaires se comportant comme des greffons autologues pérennes.

La couche aragonitique nacrée des mollusques bivalves choisis dans le groupe comprenant Pinctada Maxima, Pinctada Margaritifera, Tridacnae Maxima, Tridacnae Gigas et autres Pinctadines, est un matériau composite organique et inorganique d'origine biogène, de structure hybride. En effet, la couche aragonitique nacrée de ces mollusques bivalves se présente sous la forme d'une architecture feuilletée, alternant une composante minérale constituée de nano-cristaux d'aragonite, carbonate de calcium cristallisé dans le système orthorhombique, organisés en tablettes, et une composante organique constituée de bio-polymères linéaires et branchés, organisés en un treillis tridimensionnel. Cet ensemble confère au biomatériau une architecture lamellaire particulièrement adaptée à l'absorption et à la répartition des forces et des chocs s'opposant à la rupture.

Il a été démontré que la ténacité d'une structure lamellaire est liée à la composante organique et la rigidité à la composante minérale, et que les polymères présentent la structure idéale pour absorber et dissiper l'énergie de rupture. Toutefois, le processus d'élaboration et de croissance des valves, notamment de la couche aragonitique nacrée, peut être l'objet d'altérations dues à des facteurs endogènes tels que la physiologie et la physiopathologie, différentes d'un mollusque à l'autre, ainsi qu'à des facteurs exogènes tels que le biotope, les variations du milieu marin, la température de l'eau, la composition du zoo- et du phytoplancton, les agressions des agents pathogènes et des prédateurs.

Il en résulte une altération de l'agencement architectural macro-, micro- et nanométrique des composants, et par conséquent de la qualité de l'aragonite, avec un retentissement sur les propriétés mécaniques qui, de ce fait, ne sont pas reproductibles d'une valve à l'autre.

On a pu mettre en évidence en ingénierie qu'un agencement de structures selon un rapport de 10/1, c'est-à-dire un ratio de 400 nanomètres de structures dures pour 40 nanomètres de structures souples, constituait la référence pour la réalisation de structures lamellaires hybrides, donnant naissance à la réalisation de matériaux synthétiques bio-inspirés de type aragonitique, reproduisant l'organisation alternée d'interfaces organiques-inorganiques, pour aboutir à un matériau tridimensionnel comme l'aragonite marine des mollusques cités plus haut.

D'autre part, la particularité du treillis organique, séparant et soudant les tablettes inorganiques d'aragonite, réside dans le fait qu'il comporte des pores interconnectés de diamètres différents, communiquant dans toute son épaisseur, lui conférant une porosité continue et une porosité ouverte avec des pores débouchants.

L'os est un matériau viscoélastique dont le caractère visqueux est dû à la présence des fluides interstitiels qui l'imprègnent, et surtout à celle des bio-polymères tels que les collagènes, les glycosaminoglycanes et les protéoglycanes qui entrent dans sa composition.

Les propriétés viscoélastiques sont plus importantes chez un os cortical frais, c'est-à-dire imprégné de fluides interstitiels (plasma, sérum, etc...) que chez l'os sec.

Il en est de même de la couche aragonitique nacrée des mollusques, objet de l'invention, qui, même sèche, contient 2 à 3% d'eau, principalement localisée dans les feuillets bio-polymériques qui la composent.

Dans le brevet FR N° 09 54066 et le brevet US N° 8 485 458, il a été décrit que la fraction organique de la couche aragonitique nacrée des mollusques bivalves choisis dans les groupes comprenant Pinctada Maxima, Pinctada Margaritifera, Tridacnae Maxima, Tridacnae Gigas et autres Pinctadines, contient des molécules diffusibles, solubles, ayant des propriétés ostéogènes qui interviennent dans la croissance et la minéralisation des tissus calcifiés. Elle contient également des bio-polymères composés en grande partie de collagènes de type I et II, de glycoprotéines de faible poids moléculaire, dont certaines sont apparentées à des facteurs de croissance, à des cytokines et à d'autres molécules ostéo-compétentes intervenant dans la régénération osseuse et/ou cartilagineuse. Cette fraction organique contient également la quasi-totalité des acides aminés et notamment l'arginine, la glycine, l'acide aspartique, molécules ayant des propriétés chimio-tactiques favorisant l'adhésion cellulaire, ainsi que des métalloenzymes, métalloporphyrines, métalloprotéines, molécules intervenant dans de nombreuses réactions métaboliques lors de l'ostéogénèse. La fraction minérale, outre le carbonate de calcium, contient également de nombreux minéraux, ainsi que des métaux intervenant dans la biosynthèse des tissus calcifiés.

Les inventeurs ont démontré (C.R. Acad. Sc. Paris 1988, C.R. Acad. Sc. Paris 1989, CLINICAL MATERIAL 0267-6605/90/S03-50 1990), que le biomatériau se pérennisait en site endo-osseux, réalisant une soudure sans transition avec l'os receveur. De plus, les molécules actives contenues dans l'aragonite des mollusques cités précédemment ne présentent aucun effet cytotoxique, aucun effet mutagène, ni systémique, leur interaction n'ayant qu'un impact de potentialisation et de stimulation des facteurs locaux de la cicatrisation et de la régénération osseuses.

On a pu démontrer en biomécanique, c'est-à-dire à l'étude des propriétés mécaniques des os, qu'il y avait une relation étroite entre leur structure tridimensionnelle, leur positionnement anatomique et leurs fonctions. Les réponses spatio-temporelles sont donc adaptées aux forces et aux contraintes internes et externes, les charges appliquées sur les structures osseuses étant différentes selon leurs fonctions et leur positionnement anatomique.

Les propriétés mécaniques des os sont donc différentes selon leurs formes, leurs fonctions et leur appartenance aux différents étages musculo-squelettiques. C'est ainsi que les valeurs des différents types de mesure de résistance tels que module d'Young, contrainte à la rupture en flexion ou compression, déformation à la rupture, varient selon qu'ils sont appliqués aux os longs, tels que le fémur, le tibia, la fibula, l'humérus, le radius et le cubitus, aux os courts tels ceux du carpe, métacarpe, du tarse et du métatarse, aux massifs osseux tels que les corps vertébraux, les os du bassin, et aux os plats tels ceux de la face et du crâne, ainsi que de la clavicule et de l'omoplate.

On sait par ailleurs que le comportement de l'interface os-plaque d'ostéosynthèse ou implant est non linéaire, et que les charges appliquées sont dynamiques. C'est ainsi que le comportement d'un dispositif d'ostéosynthèse sera différent selon le positionnement anatomique de l'os concerné.

On a également pu mettre en évidence que l'aragonite des valves des mollusques cités en référence, présente des propriétés mécaniques, dont les paramètres de mesure (module d'Young, contrainte à la rupture en flexion ou en compression) ont des valeurs qui varient de manière importante et clairement identifiée selon l'origine géographique, le biotope, les variations du milieu marin, la température de l'eau, la composition du zoo- et du phytoplancton. On peut observer qu'il y a concordance entre l'échelle de diversité des valeurs de ces paramètres et celle des propriétés mécaniques de l'os, selon sa fonction et son positionnement, comme exposé précédemment.

C'est la raison pour laquelle les inventeurs proposent pour la fabrication de dispositifs d'ostéosynthèse et/ou d'implants en aragonite des valves des mollusques cités en référence, de sélectionner ces dernières en fonction de leur origine et des conditions de culture, parmi les populations présentant des valeurs de paramètres mécaniques compatibles et adaptées aux destinations des dispositifs d'ostéosynthèse et implants envisagés.

Les inventeurs ont mis en évidence que l'activité ostéoclastique, à l'origine du remaniement osseux, s'exerçait de la même manière à l'encontre du biomatériau, à l'interface os-biomatériau, mais était limitée dans le temps et couplée à l'activité concomitante d'apposition d'os néoformé par les ostéoblastes. En d'autres termes, ce phénomène biologique confirme le caractère ostéomimétique du biomatériau et explique qu'en site endo-osseux, il se soude à l'os receveur.

Physiologiquement, on observe une libération et une stimulation, in situ, de tous les signaux cellulaires et molécules nécessaires à l'induction de la cascade biologique intervenant dans la réorganisation du tissu hôte lésé.

Par ailleurs, dans le domaine odontostomatologique où les pertes de substance sont fréquentes après extraction, ainsi que dans les parodontopathies où on est amené à proposer l'utilisation de substitut osseux, la technique consiste à utiliser des membranes résorbables ou non, ce qui nécessite des vis de contention, la plupart du temps en titane ou en acier inox, afin de les maintenir et d'aménager un espace nécessaire à la protection du substitut osseux sous-membranaire. On sait que le maintien d'un matériau de comblement osseux sur le site est difficile, surtout s'il est sous forme de granules, car il est expulsé à travers le trait d'incision ou la fibro-muqueuse. Afin de maintenir en place le matériau de comblement et la membrane, on utilise des mini-vis en titane de manière à aménager un espace au-dessus de la zone à combler. Le protocole passe par une dépose de ces vis après cicatrisation et régénération osseuse avec ablation de la membrane si non résorbable.

De même en implantologie orale, où la majorité des implants sont en oxyde de titane et en zirconium, on observe les mêmes manifestations biologiques qu'avec les matériels d'ostéosynthèse et les vis de fixation, manifestations évoquées précédemment.

Pour ces raisons, les inventeurs ont mis au point un nouveau matériau hybride semi-synthétique permettant la réalisation de dispositifs d'ostéosynthèse et d'implants, destinés à être maintenus de manière pérenne sur le site fracturaire, tels que : plaques et vis d'ostéosynthèse, coins d'ostéotomie, diabolos, cales et cages intersomatiques, clous centromédullaires, têtes humérales et fémorales, cavités glénoïdes, plateaux tibiaux, condyles fémoraux, corps vertébraux, hémi-maxillaires, os de la chaîne des osselets, ancres chirurgicales pour la réinsertion ligamentaire et/ou tendineuse, gouttières gainantes pour la réduction ostéosynthésée de fractures comminutives à petits fragments, ainsi que vis de contention membranaire et implants dentaires se comportant comme des greffons autologues pérennes.

Ainsi l'invention porte sur un matériau hybride semi-synthétique qui est la couche aragonitique nacrée de mollusques bivalves choisis dans le groupe comprenant Pinctada Maxima, Pinctada Margaritifera, Tridacnae Maxima, Tridacnae Gigas et autres Pinctadines, ledit matériau hybride semi-synthétique comprenant une fraction inorganique et une fraction organique réticulée et ayant un pH de 7 à 7,4. La présente invention concerne aussi le procédé d'obtention de ce matériau par modification structurale d'un biomatériau hybride naturel qui est la couche aragonitique nacrée de mollusques bivalves choisis dans le groupe comprenant Pinctada Maxima, Pinctada Margaritifera, Tridacnae Maxima, Tridacnae Gigas et autres Pinctadines. La présente invention concerne également les procédés de fabrication de dispositifs d'ostéosynthèse et d'implants.

### Description détaillée de l'invention

Selon un premier aspect, l'invention porte sur un matériau hybride semi-synthétique qui est la couche aragonitique nacrée de mollusques bivalves choisis dans le groupe comprenant Pinctada Maxima, Pinctada Margaritifera, Tridacnae Maxima, Tridacnae Gigas et autres Pinctadines, ledit matériau hybride semi-synthétique comprenant une fraction inorganique et une fraction organique réticulée et présentant un pH de 7 à 7,4. Du fait de sa composition chimique, de ses propriétés structurales variables selon les caractéristiques de son lieu d'origine et de culture, le matériau hybride semi-synthétique de l'invention présente, en particulier par rapport à celles du titane ou de l'acier, des propriétés mécaniques adaptées, compatibles avec les différents types d'os cortical.

Le matériau hybride semi-synthétique présente une architecture feuilletée, c'est-à-dire une superposition en gradin de fractions inorganiques et de fractions organiques. En particulier, la fraction organique ne comprend pas d'inclusions minérales.

Le matériau hybride semi-synthétique n'est pas obtenu par synthèse chimique mais par modification des propriétés texturales et structurales de la couche aragonitique nacrée de mollusques bivalves choisis dans le groupe comprenant Pinctada Maxima, Pinctada Margaritifera, Tridacnae Maxima, Tridacnae Gigas et autres Pinctadines, en particulier une modification du pH et une réticulation de la fraction organique.

Le pH du matériau hybride semi-synthétique de l'invention est mesuré, sur une aliquote du matériau réduit en poudre et dispersé dans de l'eau. Le pH du matériau hybride semi-synthétique de l'invention est voisin du pH des fluides biologiques et du milieu interne (qui se situe aux environs de 7,4), ce qui permettra aux dispositifs d'ostéosynthèse et/ou implants fabriqués à partir de ce matériau d'être bien tolérés et parfaitement bio-intégrés. Par ailleurs, un tel pH crée des conditions favorables à l'induction d'une réticulation des chaînes de bio-polymères de la fraction organique du matériau hybride.

La réticulation est caractérisée par une interconnexion multidirectionnelle des chaînes des bio-polymères linéaires et branchés constituant ladite fraction organique. La réticulation induit une forte énergie de cohésion, une augmentation de l'énergie de surface et, par conséquent, une augmentation de l'adhésivité et de l'hydrophilie du matériau hybride semi-synthétique de l'invention. Elle a également pour but d'augmenter la résistance de la fraction organique du biomatériau à l'action corrosive des fluides biologiques en diminuant sa solubilité et en s'opposant à son vieillissement et à l'action des contraintes mécaniques que subit le biomatériau.

Ces modifications structurales créent également des conditions favorables à l'imprégnation du dispositif d'ostéosynthèse et/ou implant fabriqué à partir du matériau hybride semi-synthétique de l'invention par des liquides biologiques et/ou des compositions contenant des substances pharmaceutiquement actives

L'invention a également pour objet un procédé de fabrication d'un matériau hybride semi-synthétique à partir d'un biomatériau hybride naturel, qui comprend une fraction inorganique et une fraction organique, ledit procédé comprenant une étape de modification du pH et une étape de réticulation de la fraction organique dudit biomatériau hybride.

Dans le procédé de l'invention, le biomatériau hybride naturel est la couche aragonitique nacrée de mollusques bivalves choisis dans le groupe comprenant Pinctada Maxima, Pinctada Margaritifera, Tridacnae Maxima, Tridacnae Gigas et autres Pinctadines.

Les tests mécaniques pratiqués sur des échantillons d'aragonite des mollusques bivalves concernés, portant entre autres sur le module d'Young ou la contrainte à la rupture, ont montré que ce biomatériau présente, selon son origine géographique et ses conditions de culture, un éventail large de valeurs, compatibles avec la réalisation de matériel d'ostéosynthèse et d'implants selon leur destination, c'est-à-dire le type d'os concerné.

Afin que, selon l'origine et les conditions de culture initialement retenues, les propriétés mécaniques observées du biomatériau soient conservées autour des valeurs moyennes compatibles avec son utilisation comme matériel d'ostéosynthèse et/ou implant pour un type d'os concerné, il fallait donc trouver un procédé susceptible de renforcer structuralement le biomatériau afin que ses propriétés mécaniques essentielles soient conservées, homogènes selon la destination du dispositif d'ostéosynthèse et/ou implant à fabriquer.

C'est ainsi que les inventeurs proposent, compte tenu de la nature hybride feuilletée de l'aragonite des mollusques cités en référence, d'appliquer à sa composante bio-polymérique un traitement spécifique aux polymères, destiné à en modifier la structure, avec pour corollaire un renforcement de ses propriétés mécaniques.

En effet, en ingénierie et notamment dans la chimie des plastiques polymériques, il a été démontré que la réticulation consolide les propriétés mécaniques. C'est pourquoi les inventeurs proposent un procédé de réticulation, notamment par la riboflavine, des fractions collagéniques, des protéoglycanes et des glycosaminoglycanes de la fraction organique des valves des mollusques cités, en référence à la réticulation du collagène cornéen, ou cross-linking, qui est un pontage biochimique fibrillaire par des liaisons covalentes.

Afin de pouvoir utiliser le biomatériau hybride naturel pour la réalisation de dispositifs d'ostéosynthèse et/ou d'implants, les inventeurs ont trouvé qu'il était important de consolider ses propriétés mécaniques d'origine par la réticulation des chaînes des biopolymères composant sa fraction organique. Pour ce faire, il était nécessaire préalablement d'abaisser son pH, généralement compris entre 9 et 12, à un niveau compris entre 7 et 7,4, proche de celui des fluides biologiques circulants, favorisant de ce fait, la tolérance et la biointégration des dispositifs d'ostéosynthèse et/ou implants sur et dans le site receveur.

Selon un mode de réalisation particulier, l'étape de modification du pH est réalisée par immersion dans un bain d'eau du réseau microbiologiquement maîtrisée, portée à ébullition, par exemple dans un mélange à parts égales d'eau du réseau microbiologiquement maîtrisée et d'eau osmosée, jusqu'à l'obtention du pH souhaité. Le traitement est par exemple de 60 à 180 minutes, et de préférence de 60 à 120 minutes. Le pH peut être mesuré sur une aliquote du biomatériau réduit en poudre et dispersé dans l'eau.

Selon un mode de réalisation particulier, l'étape de réticulation est réalisée à l'aide d'un agent réticulant comme la riboflavine, la vitamine C, un polyol tel que le mannitol, et/ou à l'aide d'agents physiques tels que les rayonnements ionisants.

L'invention a également pour objet un procédé de fabrication d'un dispositif d'ostéosynthèse ou implant en matériau hybride semi-synthétique tel que défini précédemment. Ledit procédé comprend les étapes suivantes:
a) Sélection de valves, ayant auparavant subi l'exposition de leur couche aragonitique nacrée, des mollusques choisis dans le groupe comprenant Pinctada Maxima, Pinctada Margaritifera, Tridacnae Maxima, Tridacnae Gigas et autres pinctadines,
b) Découpe de préforme et fabrication du matériel d'ostéosynthèse, éventuellement après modélisation numérique,
c) Modification du pH et réticulation, et
d) Modification de l'état de surface,
l'ordre des étapes b) et c) étant indifférent.

L'étape a) consiste à sélectionner selon son origine et ses conditions de culture mais également selon la destination du matériel d'ostéosynthèse envisagé, une valve de mollusques qui soit d'une épaisseur et d'une intégrité physique et structurale satisfaisantes pour permettre d'y découper dans la couche aragonitique nacrée préalablement exposée, une préforme de taille adaptée au dispositif envisagé.

Afin d'exposer la couche aragonitique nacrée, le périostracum et la couche calcitique prismatique externe sont meulés par abrasion, notamment à l'aide d'une meule diamantée à grains fins, par exemple à la vitesse de 3000 tr/min sous courant d'eau. L'épaisseur est mesurée à l'aide d'un compas d'épaisseur. L'intégrité physique et structurale de la couche aragonitique de la valve sélectionnée est contrôlée dans une chambre optique, par exemple à l'aide d'une source lumineuse halogène de 500 watts

La valve est ensuite brossée, lavée à l'eau courante du réseau microbiologiquement maîtrisée à la température de 55°C.

L'étape b) de découpe et fabrication consiste, dans une première phase, à découper des préformes destinées à réaliser des dispositifs d'ostéosynthèse selon des contours préalablement imprimés, sur l'une ou l'autre des faces de la valve, aux dimensions desdits dispositifs.

Selon un mode de réalisation approprié, cette phase de découpe de l'étape b) est réalisée au jet d'eau chargée en abrasif. La découpe au jet d'eau chargée a l'avantage de ne pas générer de vibrations pouvant engendrer des amorces de micro-fractures, ni de réactions exothermiques susceptibles de provoquer une dégradation du matériau hybride.

De façon avantageuse, l'abrasif sera constitué de grains d'aragonite, empêchant ainsi toute contamination du matériau par un matériau d'une autre nature.

Par exemple, la découpe peut être réalisée de la manière suivante : ladite valve est placée dans des cadres de contention appropriés, fixés sur le tapis d'une machine de découpe, par exemple à 5 axes, à jet d'eau chargée en abrasif. L'abrasif peut être constitué de grains d'aragonite d'une granulométrie comprise entre 0,1 et 200 µm. L'eau chargée de grains d'abrasif est pulsée sous une pression de 4 000 à 6 200 bars, à l'aide de canons de focalisation de 0,50 à 1,2 mm et de buses de coupe de 0,12 à 0,40 mm de diamètre.

La seconde phase de l'étape b) concerne la fabrication par rectification ou décolletage des dispositifs d'ostéosynthèse et/ou implants, après éventuellement modélisation numérique permettant l'exploitation de ces données par des machines-outils à commande numérique.

Selon un mode de réalisation particulier, on procède à la modélisation numérique de la zone d'insertion éventuelle d'après pièces anatomiques, afin que la géométrie de la face interne du dispositif d'ostéosynthèse ou implant à fabriquer s'adapte au plus près à la topographie de cette zone. Le dispositif d'ostéosynthèse ou implant est donc réalisé de façon homologue pour membre droit ou gauche.

Selon un autre mode de réalisation particulier, des dessins et croquis des dispositifs sont numérisés afin de permettre leur fabrication par décolletage ou rectification.

La phase de fabrication par rectification ou décolletage de l'étape b) peut être réalisée, selon un mode particulier, au moyen de machines-outils à commande numérique utilisant des instruments abrasifs, diamantés ou en céramique.

Selon un autre mode de réalisation particulier, pour les dispositifs concernés, la phase de rectification plane de l'étape b) peut être réalisée à l'aide d'une machine-outil à micro-découpe au jet d'eau chargée.

L'étape c) de modification de pH et de réticulation consiste à modifier les propriétés physiques et chimiques du matériau hybride.

Selon un mode de réalisation particulier la modification du pH lors de l'étape c) est réalisée par immersion dans un bain d'eau du réseau microbiologiquement maitrisée, portée à ébullition, par exemple dans un mélange à parts égales d'eau du réseau microbiologiquement maitrisée et d'eau osmosée, jusqu'à obtention du pH souhaité. Le traitement peut par exemple être de 60 à 180 minutes, et de préférence de 60 à 120 minutes.

Selon un mode de réalisation particulier, la réticulation lors de l'étape c) est réalisée à l'aide d'un agent réticulant tel que la riboflavine, la vitamine C ou un polyol tel le mannitol. Classiquement la réticulation est réalisée à une température supérieure à 20° C. Dans le cas d'utilisation de la riboflavine, l'imprégnation du biomatériau hybride avec la riboflavine pourra avantageusement être suivie d'une exposition actinique aux UVA. La réticulation peut également être obtenue par l'utilisation d'agents physiques tels que les rayonnements ionisants.

Les inventeurs ont privilégié l'action de la riboflavine, ou vitamine B2, en tant qu'agent réticulant en raison de ses propriétés pharmacologiques, biochimiques et physiques. En effet la riboflavine supporte très bien la stérilisation et la congélation, et stimule le métabolisme cellulaire. Considérée comme un facteur de croissance cellulaire, elle intervient dans la synthèse des protéines, des glucides et des lipides et possède des propriétés anti-oxydantes puissantes, qui s'opposent à l'action des radicaux libres produits par l'action actinique lors de la réticulation. On voit donc que l'utilisation de la riboflavine hydrosoluble a pour effet non seulement d'en modifier la structure, mais de conférer au matériau semi-synthétique de nouvelles propriétés pharmacologiques intéressant la régénération et la cicatrisation osseuses.

L'étape d) consiste à modifier l'état de surface dudit dispositif d'ostéosynthèse ou implant en appliquant quatre traitements successifs de sablage, nettoyage par ultrasons, cryogénie et application de nanoparticules. L'objectif est d'une part d'améliorer les propriétés tribologiques, notamment les liaisons fortes de type covalentes à l'origine de l'agrégation des nanoparticules et de leur interaction avec le milieu receveur, d'autre part d'augmenter le rapport surface/volume entre le dispositif et l'os cortical fracturé pour en assurer la stabilité, enfin, de favoriser la libération des molécules solubles ostéogénitrices, activatrices des facteurs locaux de l'ostéogénèse qui y sont contenus.

Selon un mode de réalisation particulier le traitement de sablage de l'étape d) consiste à modifier l'état de surface du dispositif d'ostéosynthèse ou implant afin d'en améliorer l'ancrage sur l'os.

Par exemple, il peut être réalisé de la façon suivante : ledit dispositif est placé dans une sableuse à manche et traité par des projections successives, à l'aide d'un système de surpression, préférentiellement de grains d'aragonite de 25 à 70 µm propulsés à l'aide de buses de sablage rondes de 0,8 mm, et de grains d'aragonite de 70 à 250 µm propulsés à l'aide de buses de 1,2 mm, sous une pression de 6 bars.

Selon un mode de traitement particulier, le nettoyage par ultrasons de l'étape d) se fait comme suit : une cuve à ultrasons est remplie d'eau chaude du réseau microbiologiquement maîtrisée, à 55°C, température d'efficacité maximale, jusqu'à un repère indiquant le volume d'eau souhaité. Celle-ci est additionnée d'une solution nettoyante et désinfectante à une dilution de 1 :128, soit 1 part de solution pour 127 parts d'eau. Après 15 minutes de dégazage destiné à éliminer les bulles d'air, le dispositif d'ostéosynthèse ou implant est placé dans la cuve pour une durée de 30 minutes à une fréquence de 40 kHz pour une cavitation induisant un retrait particulaire optimum.

Ledit dispositif est ensuite rincé sous courant d'eau du réseau microbiologiquement maîtrisée, pendant 20 minutes, puis immergé pendant 20 minutes dans un bain d'eau déminéralisée à une température de 90°C, additionnée de 2% d'eau de Javel à 2,6% de chlore actif pendant 30 minutes, puis rincé à nouveau à l'eau déminéralisée à 90°C.

Enfin, le dispositif est mis à tremper dans de l'eau déminéralisée à 50°C, additionnée de Calbénium® liquide, ou tout autre agent biocide, virucide, tensioactif, dilué à 2% pendant 30 minutes, rincé, puis séché.

Selon un mode de réalisation particulier, le traitement par cryogénie non abrasive de l'étape d), destiné à préparer les faces du dispositif en contact avec l'os cortical, consiste à projeter sur celles-ci des petites billes de glace sèche d'azote liquide à -80°C, de 1 mm de diamètre. L'objectif est d'optimiser l'état de surface par effet mécanique associé à un choc thermique, du fait de la différence de température entre la surface à traiter et les billes d'azote liquide lors de la sublimation de celles-ci à l'impact. La mise en oeuvre consiste à projeter dans un espace dédié sur la ou les surfaces à traiter un mélange air comprimé-billes de glace, sous une pression permettant un traitement non abrasif optimisé par la faible dureté de la glace sèche d'azote qui est 2 Mohs.

Selon l'invention, la phase application de nanoparticules de l'étape d) de modification de l'état de surface est un revêtement de nanoparticules mécano-structurées obtenues à partir du biomatériau hybride, selon le brevet FR N° 09 54066 et le brevet US N° 8 485 458.

Cette phase de l'étape d) de modification de l'état de surface est réalisée, soit par immersion, dans une émulsion de viscosité variable, desdites nanoparticules mécano-structurées, soit par centrifugation, soit par pulvérisation et soit, de manière préférentielle, par électrodéposition, qui consiste à plonger les dispositifs d'ostéosynthèse et les implants dans un bain électrolytique desdites nanoparticules de manière à initier une électrodéposition de celles-ci sur la surface de ces derniers.

A l'issue des traitements physiques et chimiques appliqués selon le procédé de l'invention, on peut considérer que l'aragonite des mollusques concernés, constitutive des dispositifs fabriqués selon l'invention, a été transformée en matériau hybride semi-synthétique.

Selon un mode de réalisation particulier, le procédé de fabrication d'un dispositif d'ostéosynthèse et/ou implant comprend en outre une étape e) d'imprégnation de celui-ci par des liquides biologiques et/ou des compositions contenant des substances pharmaceutiquement actives.

Afin d'uniformiser les propriétés viscoélastiques des dispositifs d'ostéosynthèse et implants fabriqués selon l'invention, les inventeurs proposent ainsi d'imprégner les bio-polymères qui entrent dans leur composition avec des plasmas ou sérums des différents systèmes antigéniques des groupes sanguins, par trempage et/ou imbibition, à pression atmosphérique ou sous vide, ce qui, en outre, augmente leur bio-acceptation.

Dans un autre mode de réalisation préférentiel, les dispositifs et implants peuvent être imprégnés par des substances médicamenteuses telles que des anti inflammatoires non stéroïdiens, antalgiques, antibiotiques et antimitotiques ou toute autre substance à effet thérapeutique.

Selon un mode de réalisation particulier, le procédé de fabrication d'un dispositif comprend en outre des phases de conditionnement, par exemple, sous double emballage, de stérilisation, sous atmosphère protectrice, réalisée à l'aide de rayonnements ionisants à 25 KGy, ainsi qu'une étape de stockage, soit à une température de 0° à 4°C ou par congélation à -15°C, soit, pour les dispositifs n'ayant pas fait l'objet d'imbibition par les liquides biologiques, un stockage à température ambiante

Les dispositifs peuvent aussi être imprégnés extemporanément de sang total ou de plasma autologues au temps de l'intervention.

L'invention a également pour objet un dispositif en matériau hybride semi-synthétique, ou obtenu selon le procédé de fabrication dudit dispositif. Ledit dispositif est choisi parmi des plaques d'ostéosynthèse, telles que plaque d'ostéosynthèse droite, plaque d'ostéosynthèse épiphyso-diaphysaire, plaque d'ostéosynthèse pour fracture malléolaires, plaque ruptive d'ostéosynthèse épiphyso-diaphysaire, vis d'ostéosynthèse, vis de contention membranaire, coins d'ostéotomie, diabolos, cales et cages inter-somatiques, clous centromédullaires, têtes humérales et fémorales, cavités glénoïdes, plateaux tibiaux, condyles fémoraux, corps vertébraux, hémi-maxillaires, chaine des osselets, ancres chirurgicales pour la réinsertion ligamentaire et tendineuse, gouttières gainantes pour la réduction ostéosynthésée de fractures comminutives à petits fragments et implants dentaires.

Selon un mode de réalisation particulier, le dispositif comporte au moins un moyen de contention qui, lors de la pose dudit dispositif s'oppose à son déplacement, ledit moyen étant choisi parmi des ergots, des encoches, un harpon claveté et aplati, et un filetage s'opposant au dévissage.

Selon le mode de réalisation particulier où le moyen de contention est constitué d'ergots, ces derniers, diamétralement opposés, permettent de verrouiller ledit dispositif sur les fragments osseux de part et d'autre du trait de fracture après aménagement dans la corticale, à l'aide de plaque fantôme, des puits d'insertion des ergots, ce qui réalise de ce fait une fixation primaire immédiate en clavette et une fixation secondaire, une fois la cicatrisation osseuse réalisée.

Selon le mode de réalisation particulier où le moyen de contention est constitué par des encoches rectilignes transversales en marche d'escalier, celles-ci, de par leur géométrie, s'opposent au glissement vers l'avant ou vers l'arrière dudit dispositif.

Selon le mode de réalisation particulier où le moyen de contention est un harpon claveté aplati, les caractéristiques de ce dernier s'opposent au glissement et à la rotation dudit dispositif.

Selon le mode de réalisation particulier où le moyen de contention est un filetage, ce dernier présente une géométrie de pas non métrique, et de forme trapézoïdale, qui s'oppose à son dévissage.

De façon avantageuse, ce filetage favorise un comblement total des spires par le tissu osseux néoformé.

Selon un mode de réalisation approprié, le dispositif comprend en outre des trous de fixation. Le nombre, la taille et l'emplacement de ces trous de fixations seront bien entendu adaptés par l'homme du métier en fonction de la forme et des dimensions du dispositif.

Lesdits trous de fixation peuvent être fraisés, partiellement ou entièrement et, éventuellement, être taraudés d'un filetage métrique iso à pas standard ou à pas fin.

Selon un mode de réalisation approprié, lesdits trous de fixation peuvent être ronds ou oblongs et/ou faire un angle aigu avec la verticale.

Selon un mode de réalisation particulier, le dispositif comprend en outre un moyen qui, lors de la pose dudit dispositif permet son ajustement, de façon à s'adapter au mieux à la morphologie du site fracturaire. Selon un mode réalisation approprié, le moyen qui, lors de la pose du dispositif, permet son ajustement, est un renflement positionné sur la face interne dudit dispositif de hauteur comprise entre 0,1 et 0,5 mm, de préférence comprise en 0,15 et 0,25 mm. Ce renflement pourra être meulé à la demande juste avant la pose.

Selon un mode de réalisation particulier, le dispositif, qui est une plaque ruptive d'ostéosynthèse épiphyso-diaphysaire, comprend en outre deux encoches de rupture en forme de V, l'une sur la face externe, l'autre sur la face interne. L'encoche de rupture de la face externe du dispositif est parfaitement repérable à la palpation et indique le siège de l'incision cutanée à effectuer. Ledit dispositif peut alors être rompu à l'aide d'un marteau et d'un burin chirurgical, permettant au membre de poursuivre son développement, les deux parties dudit dispositif s'éloignant au fur et à mesure de l'évolution du cartilage de conjugaison.

Lors des interventions, tous les matériels d'ostéosynthèse fabriqués selon l'invention, sont posés à l'aide d'ancillaires, comprenant des plaques fantômes réalisées aux dimensions et aux caractéristiques des dispositifs d'ostéosynthèse et comportant entre autres les accessoires suivants : un ou plusieurs canons de perçage, des jauges de profondeur et de maintien, des forêts et des tarauds aux dimensions des vis, des porte-vis ainsi que des porte-plaques, des daviers de maintien des fragments, des tournevis et des clefs pour têtes de vis hexagonales, des distracteurs, des cales d'ouverture, des cales de maintien, ..

Les exemples suivants illustrent l'invention sans qu'ils soient limitatifs et il appartiendra à l'homme de métier de mettre en oeuvre tous les procédés de l'invention à chaque fois qu'il souhaitera réaliser un dispositif d'ostéosynthèse ou un implant.

### Exemples

### Exemple 1: Procédé de fabrication de dispositifs d'ostéosynthèse

On choisit des valves de mollusque, dans cet exemple, Pinctada Maxima, dont l'épaisseur, mesurée à l'aide d'un compas d'épaisseur, est suffisante pour la réalisation des dispositifs d'ostéosynthèse souhaités.

Le périostracum et la couche calcitique prismatique externe sont meulés par abrasion à l'aide d'une meule diamantée à grains fins, à la vitesse de 3000 tr/min sous courant d'eau, ce qui permet d'en exposer la couche aragonitique nacrée.

L'intégrité physique et structurale des valves choisies est contrôlée dans une chambre optique à l'aide d'une source lumineuse halogène de 500 watts.

Les valves sont ensuite brossées, lavées à l'eau courante du réseau microbiologiquement maîtrisée à la température de 55°C.

Afin d'obtenir des préformes aux dimensions calculées selon celles des dispositifs d'ostéosynthèse que l'on veut réaliser, les contours sont imprimés sur l'une ou l'autre des faces des valves sélectionnées. Les valves sont ensuite découpées suivant les contours dessinés. Pour ce faire, les valves sont placées dans des cadres de contention appropriés, fixés sur le tapis d'une machine de macro-découpe 5 axes, à jet d'eau chargée en abrasif, par exemple de grains d'aragonite d'une granulométrie de 150 µm environ, pulsée sous une pression de 4 135 à 6 150 bars, à l'aide de canons de focalisation de 0,50 à 1,2 mm et de buses de coupe de 0,20 à 0,40 mm de diamètre.

On obtient ainsi des préformes brutes.

Afin que la géométrie de la face interne du dispositif d'ostéosynthèse s'adapte au plus près à la topographie de sa zone d'insertion éventuelle, on procède à la modélisation numérique de cette dernière sur pièce anatomique, ce qui permet de décliner de façon homologue les dispositifs d'ostéosynthèse pour membre droit ou gauche.

Les formes et les dimensions des dispositifs d'ostéosynthèse font également l'objet à partir d'ébauches, d'une modélisation numérique. Ces données numériques sont ensuite utilisées afin de permettre la fabrication par rectification ou décolletage des dispositifs d'ostéosynthèse à l'aide de machines à commande numérique.

La rectification des pièces d'ostéosynthèse, hors vis, peut être réalisée par l'application d'un process de meulage sous courant d'eau, par instruments rotatifs abrasifs diamantés ou en céramique.

Les vis d'ostéosynthèse peuvent quant à elles être obtenues par un process de décolletage sous courant d'eau, à l'aide d'instruments rotatifs abrasifs diamantés ou en céramique.

Les dispositifs d'ostéosynthèse sont ensuite plongés dans un mélange à parts égales d'eau du réseau microbiologiquement maitrisée, et d'eau osmosée, porté à ébullition à 100°C, pour une durée variable selon leur épaisseur, par exemple 60 minutes, afin d'amener le pH du biomatériau hybride constitutif à une valeur comprise entre 7 et 7,4.

Puis, les dispositifs d'ostéosynthèse sont immergés dans une solution de riboflavine à 5 % pendant 48 heures, à une température supérieure à 20° C, afin de réaliser le protocole de réticulation des chaînes de bio-polymères de la fraction organique du biomatériau hybride constitutif. Les dispositifs sont ensuite rincés, puis placés dans une enceinte en verre, munie de lampes UVA d'une longueur d'onde de 365 nm, sous une intensité de 2300 µJ/cm², pendant 20 minutes. Les dispositifs d'ostéosynthèse sont ensuite séchés par courant d'air chaud à 40°C.

Au microscope électronique à balayage on observe une densification du réseau du treillis bio-polymérique du matériau constitutif.

Afin de favoriser l'ancrage des dispositifs d'ostéosynthèse réalisés, l'état de surface des parties destinées à être en contact avec l'os est modifié. On procède dans un premier temps au sablage des faces et des bords dans une sableuse à manche, par système de surpression, successivement avec des buses de sablage rondes de 0,8 mm, par des grains d'aragonite de 25 à 70 µm, puis des buses de 1,2 mm, par des grains d'aragonite de 70 à 250 µm, sous une pression de 6 bars.

Les dispositifs d'ostéosynthèse font ensuite l'objet d'un traitement aux ultrasons, comme suit. Une cuve à ultrasons est remplie d'eau chaude du réseau microbiologiquement maîtrisée, à 55°C, température d'efficacité maximale, jusqu'à un repère indiquant le volume d'eau souhaité. Celle-ci est additionnée d'une solution nettoyante et désinfectante à une dilution de 1 :128, soit 1 part de solution pour 127 parts d'eau. Après 15 minutes de dégazage destiné à éliminer les bulles d'air, les dispositifs sont placés dans la cuve pour une durée de 30 minutes à une fréquence de 40 kHz pour une cavitation induisant un retrait particulaire optimum.

Puis les dispositifs d'ostéosynthèse sont rincés sous courant d'eau du réseau microbiologiquement maîtrisée, pendant 20 minutes, puis immergés pendant 20 minutes dans un bain d'eau déminéralisée à une température de 90°C, additionnée de 2% d'eau de Javel à 2,6% de chlore actif pendant 30 minutes, puis rincés à nouveau à l'eau déminéralisée à 90°C.

Enfin, les dispositifs d'ostéosynthèse sont mis à tremper dans de l'eau déminéralisée à 50°C, additionnée d'un agent biocide, par exemple le Calbénium® liquide, ou tout autre agent virucide ou tensioactif, dilué à 2%, pendant 30 minutes, rincés, puis séchés.

Deux traitements successifs sont ensuite effectués sur les dispositifs d'ostéosynthèse. Les faces des dispositifs qui sont destinées à être en contact avec l'os cortical subissent un traitement par cryogénie non abrasive. Ce traitement consiste à projeter sur celles-ci des petites billes de glace sèche d'azote liquide à - 80°C, de 1 mm de diamètre, de manière à optimiser l'état de surface par effet mécanique associé à un choc thermique du fait de la différence de température entre la surface à traiter et les billes d'azote liquide lors de la sublimation de celles-ci à l'impact. La mise en oeuvre consiste à projeter dans un espace dédié sur la ou les surfaces à traiter, un mélange air comprimé-billes de glace, sous une pression permettant un traitement non abrasif optimisé par la faible dureté de la glace sèche d'azote liquide qui est de 2 Mohs.

Les dispositifs sont ensuite soumis à un traitement de revêtement de nanoparticules mécano-structurées, obtenues à partir du biomatériau hybride, selon les brevets FR N° 09 54066 et US N° 8 485 458, par exemple par électrodéposition, traitement qui consiste à plonger les dispositifs d'ostéosynthèse dans un bain électrolytique des nanoparticules mécano-structurées de manière à initier une électrodéposition de celles-ci sur la surface des dispositifs d'ostéosynthèse.

Les dispositifs d'ostéosynthèse sont enfin séchés dans un courant d'air chaud à 40°C pendant 30 minutes, conditionnés sous double emballage, stérilisés, sous atmosphère protectrice à l'aide de rayonnements ionisants à 25 KGy, et stockés à la température ambiante.

### Exemple 2: Plaque d'ostéosynthèse droite

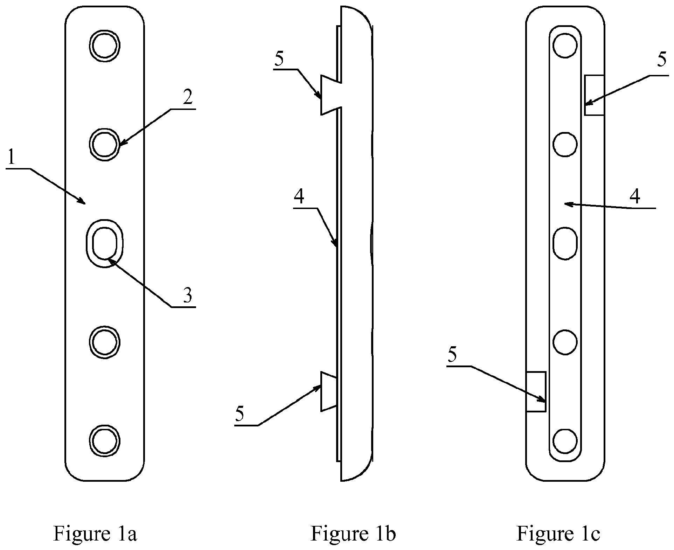

Une plaque d'ostéosynthèse droite est obtenue selon le procédé décrit dans l'exemple 1. Les Figures 1a, 1b et 1c représentent respectivement, une vue de la face externe, une vue latérale et une vue de la face interne de la plaque d'ostéosynthèse droite 1.

La plaque d'ostéosynthèse droite 1 se présente sous la forme d'un parallélépipède de longueur, de largeur et d'épaisseur variables. Sa face externe, plane, rayonnée sur tout son pourtour, est percée de plusieurs trous de fixation débouchants 2, 3, de diamètres variables, dont le plus central 3 est oblong, pour permettre une translation de la plaque en fonction de la topographie du site fracturaire.

Les trous de fixation débouchants 2, 3 sont taraudés sur la moitié inférieure de leur hauteur, d'un filetage métrique iso à pas standard ou à pas fin, correspondant au filetage sous tête des vis de fixation, et fraisés à leur moitié supérieure. Les bords externes de la plaque sont rayonnés sur tout le périmètre.

La face interne en contact avec l'os comporte sur toute sa longueur un renflement 4 arrondi d'une épaisseur maximale de 0,2 mm, englobant les trous de fixation des vis 2, 3.

Ce renflement est destiné à permettre l'ajustement de la plaque au plus près des variations anatomiques du site d'insertion. En effet, l'ostéologie montre que l'anatomie osseuse est reproductible d'un individu à l'autre ; les différences portant sur les accidents et repères anatomiques que sont les tubérosités, les tubercules, les apophyses, les gouttières, les lignes, les fossettes, dont les formes et les volumes peuvent varier de quelques dixièmes de millimètres. Aussi, compte tenu de la nature du matériau semi-synthétique, selon l'invention, il est possible en per-opératoire, de retoucher la surface du renflement à l'aide d'un instrument rotatif diamanté sous courant d'eau stérile réfrigérée, afin d'ajuster au plus près la plaque à la topographie du site d'insertion de manière à ce que l'interface os-plaque soit la plus intime possible.

La plaque d'ostéosynthèse droite 1 comporte en outre, sur sa face interne, le long de ses plus grandes dimensions, deux ergots 5, de forme trapézoïdale, de dimensions variables, l'un au tiers supérieur, l'autre au tiers inférieur, et diamétralement opposés.

La fonction de ces ergots est de verrouiller la plaque sur les fragments osseux de part et d'autre du trait de fracture après aménagement dans la corticale des puits d'insertion des ergots à l'aide de la plaque fantôme, ce qui réalise de ce fait une fixation primaire immédiate en clavette et une fixation secondaire, une fois la cicatrisation osseuse réalisée.

### Exemple 3 : Plaque d'ostéosynthèse épiphyso-diaphysaire

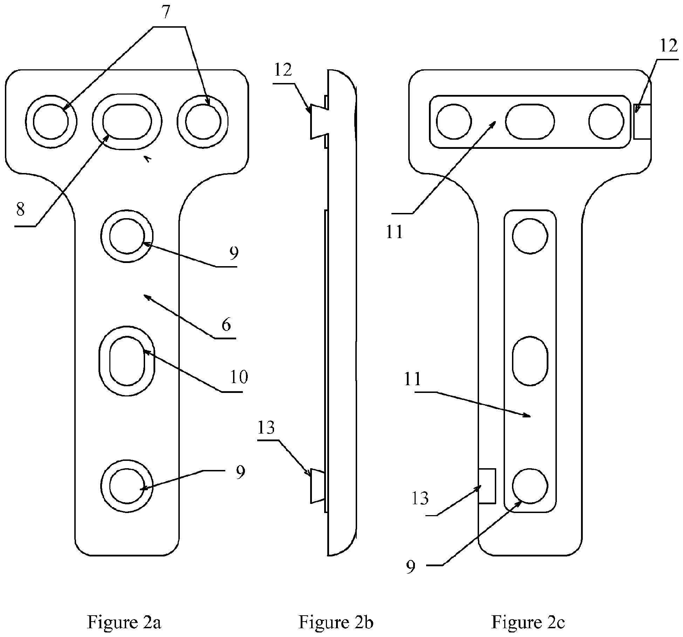

Une plaque d'ostéosynthèse épiphyso-diaphysaire est obtenue selon le procédé décrit dans l'exemple 1. Les Figures 2a, 2b et 2c représentent respectivement, une vue de la face externe, une vue latérale et une vue de la face interne de la plaque d'ostéosynthèse épiphyso-diaphysaire 6.

La plaque d'ostéosynthèse en T épiphyso-diaphysaire 6 présente une partie horizontale épiphysaire de longueur, de hauteur et d'épaisseur variables, incurvée d'arrière en avant et en dedans, de manière à épouser la topographie méta-épiphysaire.

Elle est percée de trous de fixation débouchants 7, 8 en nombre et diamètre variables (trois sur la Figure 2a), fraisés sur leur moitié supérieure et taraudés sur leur moitié inférieure au pas métrique iso standard ou fin.

Le trou de fixation central 8 de dimensions variables, est oblong, de manière à permettre une translation suivant les besoins.

La barre verticale diaphysaire, de dimensions variables, légèrement convexe de haut en bas et en dedans, et d'arrière en avant, épousant la topographie du relief diaphysaire, est percée de trous de fixation débouchants 9, 10 en nombre et diamètre variables (trois sur la Figure 2a), dont le trou central 10 est oblong pour permettre une translation si nécessaire, avant serrage des vis. Ils sont fraisés dans leur moitié supérieure et taraudés au pas métrique iso, standard ou fin sur la moitié inférieure.

Les bords externes de la plaque sont rayonnés sur tout le périmètre.

Les faces externes des barres épiphysaires et diaphysaires sont planes.

Les faces internes comportent un renflement 11 arrondi d'une hauteur maximale de 0,2 mm, englobant les trous de fixation 7, 8, 9, 10. Elles comportent aussi deux ergots 12, 13 faisant office de clavettes dans la corticale, diamétralement opposés, de forme trapézoïdale, de dimensions variables, l'un 12, sur le bord vertical postérieur de la barre épiphysaire, l'autre 13, au tiers inférieur du bord antérieur de la barre diaphysaire, s'opposant ainsi à la rotation et au déplacement des fragments distal et proximal de l'os fracturé.

### Exemple 4 : Plaque d'ostéosynthèse ruptive épiphyso-diaphysaire

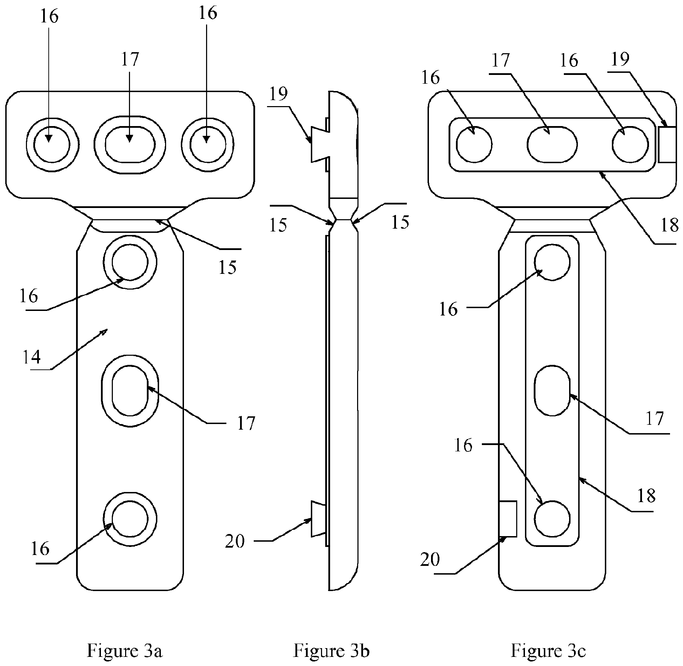

Une plaque d'ostéosynthèse ruptive épiphyso-diaphysaire est obtenue selon le procédé décrit dans l'exemple 1. Les Figures 3a, 3b et 3c représentent respectivement une vue de la face externe, une vue latérale et une vue de la face interne de la plaque d'ostéosynthèse ruptive épiphyso-diaphysaire 14.

Cette plaque ruptive est destinée à une utilisation en chirurgie pédiatrique.

La plaque d'ostéosynthèse ruptive épiphyso-diaphysaire 14 se présente sous la forme d'un T de dimensions variables, similaire dans toutes ses caractéristiques à la plaque pour adulte de l'exemple 3, et comporte en outre sur ses deux faces, à la jonction entre la barre horizontale et la barre verticale, deux encoches 15 en forme de V intéressant les bords.

Les barres horizontale et verticale sont percées de trous de fixation débouchants 16, 17 en nombre et diamètre variables, fraisés sur leur moitié supérieure et taraudés sur leur moitié inférieure au pas métrique iso standard ou fin.

Le trou de fixation central 17, des barres horizontale et verticale, de dimensions variables, est oblong, de manière à permettre une translation suivant les besoins.

Les bords externes de la plaque sont rayonnés sur tout le périmètre.

Les faces externes des barres horizontale et verticale sont planes.

Les faces internes (représentées sur la Figure 3c) de la barre horizontale et de la barre verticale comportent chacune un renflement 18 arrondi, d'une hauteur maximale de 0,2 mm, englobant les trous de fixation 16, 17. Elles comportent aussi deux ergots 19, 20 faisant office de clavettes dans la corticale, diamétralement opposés, de forme trapézoïdale, de dimensions variables, l'un 19 sur le bord vertical postérieur de la barre horizontale, l'autre 20 au tiers inférieur du bord antérieur de la barre verticale, s'opposant ainsi à la rotation et au déplacement des fragments distal et proximal de l'os fracturé.

Les encoches 15 en forme de V, situées à la jonction de la barre horizontale et de la barre verticale, permettent la rupture de la plaque en deux parties. Ces encoches seront parfaitement repérées à la palpation et indiqueront le siège de l'incision cutanée à effectuer.

La plaque pourra alors être rompue à l'aide d'un marteau et d'un burin chirurgical, permettant au membre de poursuivre son développement, les deux parties du matériel d'ostéosynthèse s'éloignant au fur et à mesure de l'évolution du cartilage de conjugaison.

### Exemple 5 : Plaque d'ostéosynthèse pour fracture malléolaires

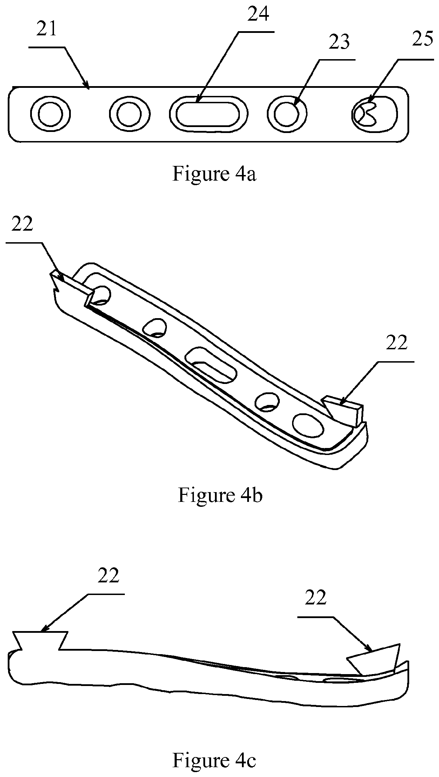

Une plaque d'ostéosynthèse pour fracture malléolaires est obtenue selon le procédé décrit dans l'exemple 1. Les Figures 4a, 4b et 4c représentent respectivement une vue de la face externe, une vue isométrique de la face interne et une vue latérale de la plaque d'ostéosynthèse pour fracture malléolaires 21.

La plaque d'ostéosynthèse pour fracture malléolaires 21 se présente sous la forme générale d'un parallélépipède de longueur et de largeur variables, dont l'extrémité inférieure est convexe d'avant en arrière et de bas en haut sur la moitié de sa longueur, et rayonnée sur tout son pourtour.

Sa face interne est concave d'arrière en avant et comporte à chaque extrémité des ergots 22 de forme trapézoïdale de dimensions variables. L'ergot situé à l'extrémité inférieure est positionné de manière à se fixer sur l'extrémité inférieure de l'apophyse styloïde, une fois la fracture réduite.

La plaque est percée de trous de fixation 23, 24, 25, en nombre variable selon sa longueur, et rayonnés sur tout son pourtour.

Le trou le plus central 24 est de forme oblongue pour permettre une éventuelle translation. Le dernier trou de fixation inférieur 25 fait un angle de 15° avec la verticale pour permettre une fixation de l'apophyse styloïde sur la partie distale de la fibula avec appui bi-cortical.

### Exemple 6 : Gouttière pour l'ostéosynthèse de fractures diaphysaires comminutives

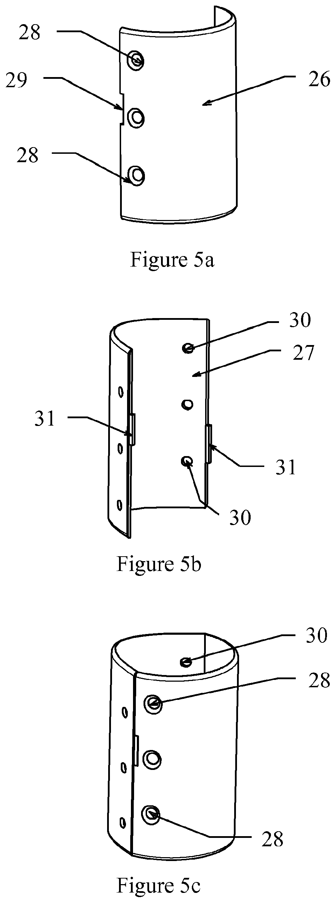

Une gouttière pour l'ostéosynthèse de fractures diaphysaires comminutives est obtenue selon le procédé décrit dans l'exemple 1. Elle est représentée sur les Figures 5a, 5b et 5c. Elle se compose de deux parties qui sont deux demi-manchons 26, 27, usinés d'après modélisation des zones éventuelles d'insertion du dispositif d'ostéosynthèse. La face externe du demi-manchon 26 est représentée sur la Figure 5a et la face interne du demi-manchon 27 sur la Figure 5b. L'ensemble de la gouttière assemblée avec les deux demi-manchons est représenté sur la Figure 5c.

Le demi-manchon 26 se présente sous la forme d'un demi-cylindre de diamètre et de longueur variables, sa face externe est convexe sur toute sa hauteur, percée de trous de fixation 28 débouchants, en nombre et diamètre variables, alignés sur les bords longitudinaux de la gouttière et décalés les uns par rapport aux autres de manière à ce que les vis de fixation se croisent.

Les trous de fixation 28 sont taraudés au pas métrique iso sur la moitié interne de leur hauteur et fraisés sur l'autre moitié. La face interne est concave sur toute sa hauteur ; elle présente le long des bords longitudinaux, deux encoches 29 diamétralement opposées, l'une au tiers supérieur, l'autre au tiers inférieur.

L'autre demi-manchon 27 est usiné d'après modélisation numérique de la zone homologue du demi-manchon 26, de section généralement triangulaire, s'adaptant à la morphologie osseuse de la zone diaphysaire considérée. Il est de longueur et d'épaisseur comparables à celles du demi-manchon 26 et est percé de trous de fixation 30 débouchants, taraudés sur toute leur hauteur au pas métrique iso correspondant au filetage de l'extrémité des vis de fixation. Ces trous sont appairés à ceux 28 du demi-manchon 26 et diamétralement opposés. Il présente le long de ses bords longitudinaux et diamétralement opposés deux ergots 31, de dimensions variables, et correspondant aux encoches 29 du demi-manchon 26.

La Figure 5c représente les deux demi-manchons assemblés, les ergots 31 du demi-manchon 27 se fixant dans les encoches 29 du demi-manchon 26.

Les deux demi-manchons 26, 27 sont biseautés, de manière à se coapter lors de l'ostéosynthèse, leurs bords supérieurs et inférieurs sont rayonnés.

### Exemple 7 : Vis d'ostéosynthèse

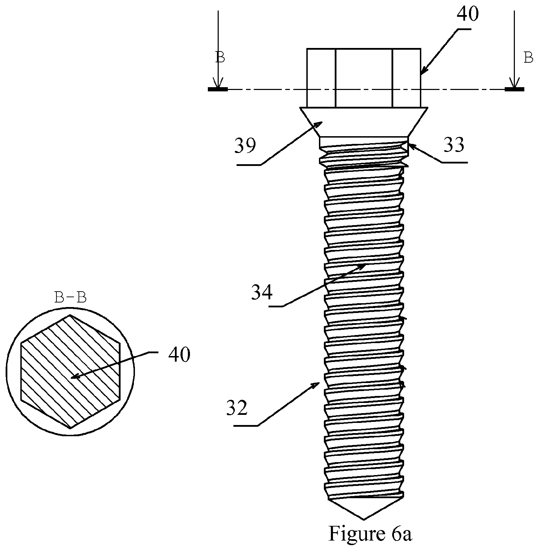

Une vis d'ostéosynthèse est obtenue selon le procédé équivalent à celui décrit dans l'exemple 1. La Figure 6a représente une vue de la vis d'ostéosynthèse 32.

La vis d'ostéosynthèse 32 se présente sous une forme générale cylindrique composée d'un filetage et d'une tête de diamètres différents et de dimensions variables.

L'extrémité supérieure 39 de la vis est fraisée, et surmontée d'un dispositif hexagonal 40 permettant un vissage avec un ancillaire adéquat.

La partie sous tête 33 est filetée dans d'un pas métrique iso, à pas standard ou à pas fin, correspondant au pas de la partie interne des trous de fixation des plaques.

La partie de la vis 34 sous le filetage métrique iso, est filetée selon un pas particulier, et d'une géométrie propre d'une part à s'opposer au dévissage et d'autre part à favoriser un comblement total des spires par le tissu osseux néoformé.

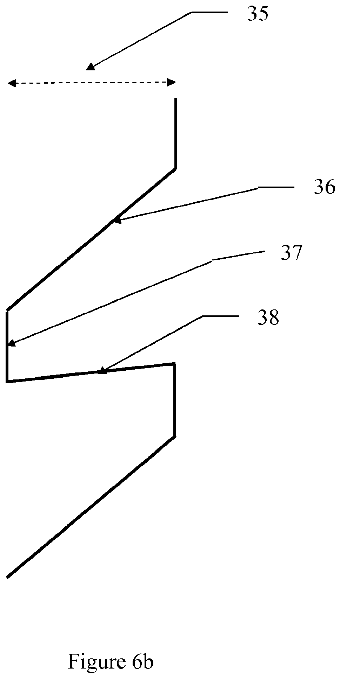

Ce pas, est représenté sur la Figure 6b. D'une profondeur 35 de 0,40 mm, il se présente sous la forme d'un trapèze quelconque, dont le côté supérieur oblique 36, de dehors en dedans et de haut en bas, présente avec l'extrémité supérieure du fond de filet 37 un angle de 135° environ et le côté inférieur oblique 38, de dehors en dedans et de haut en bas, présente avec l'extrémité inférieure du fond de filet 37 un angle de 80° environ.

### Exemple 8 : Ancre chirurgicale pour la réinsertion ligamentaire et/ou tendineuse

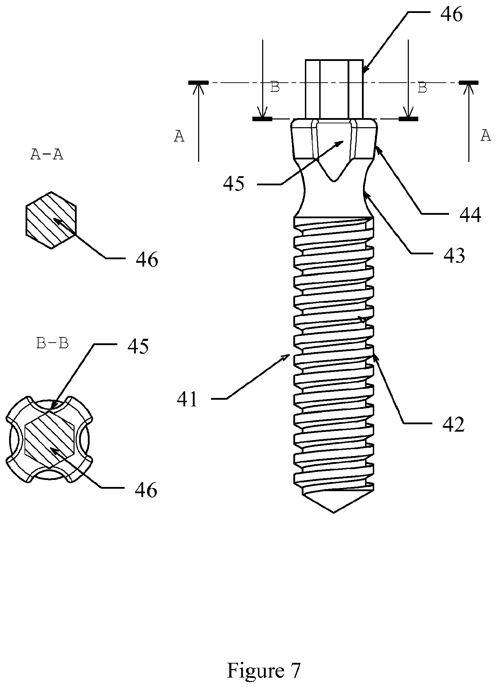

Une ancre chirurgicale pour la réinsertion ligamentaire et/ou tendineuse est obtenue selon le procédé décrit dans l'exemple 1. La Figure 7 représente une vue d'ensemble de l'ancre chirurgicale pour la réinsertion ligamentaire et/ou tendineuse 41.

L'ancre chirurgicale pour la réinsertion ligamentaire et/ou tendineuse 41 est de forme générale cylindrique et comporte sur les deux tiers inférieurs un filetage 42, tel que représenté sur la Figure 6b, s'opposant au dévissage.

Le tiers supérieur, à partir de la limite supérieure du filetage 42, comporte un étranglement circulaire 43 de profondeur et de hauteur variables, surmonté d'un quadrilatère 44 aux angles rayonnés présentant sur ses quatre faces une dépression 45 arrondie de diamètre variable.

Le quadrilatère 44 est surmonté d'une structure hexagonale 46 de hauteur variable, inscrite dans le périmètre de celui-ci.

### Exemple 9 : Cage inter-somatique

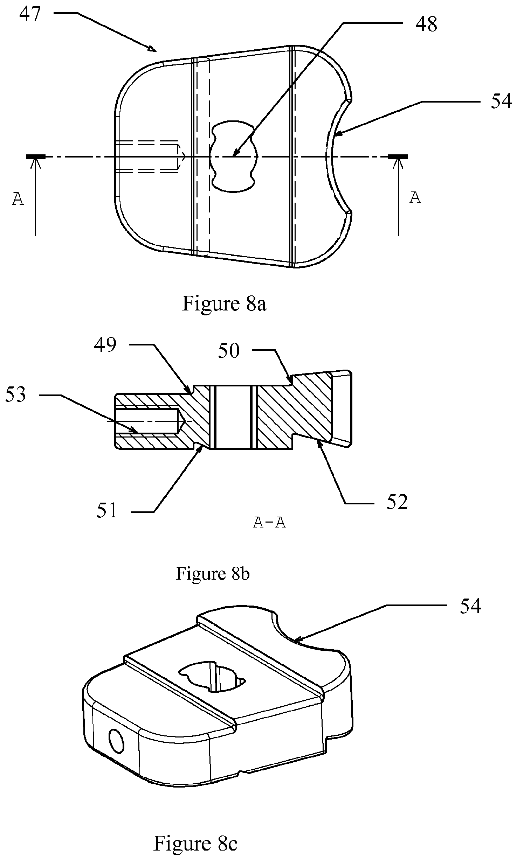

Une cage inter-somatique est obtenue selon le procédé décrit dans l'exemple 1. Les Figures 8a, 8b et 8c représentent respectivement la face inférieure, une coupe sagittale, et la face supérieure de la cage inter-somatique 47.

La cage inter-somatique 47 se présente sous la forme générale d'un parallélépipède à base trapézoïdale, aux dimensions variables, dont la partie centrale est percée d'un orifice 48 débouchant, de diamètre variable, en queue d'aronde, dans le sens transversal.

La face supérieure, plane, comporte deux encoches 49, 50 rectilignes transversales en marche d'escalier, l'une 49 à distance et en avant du bord antérieur de l'orifice central, l'autre 50 à distance et en arrière de son bord postérieur. Ces encoches s'opposent au glissement vers l'avant de la cage.

La face inférieure, plane, comporte également deux encoches 51, 52 en plan incliné de haut en bas et d'avant en arrière, l'une 51 à distance du bord antérieur de l'orifice central, l'autre 52 à distance de son bord postérieur, s'opposant au glissement vers l'arrière de la cage.

La face supérieure est usinée selon un angle prédéterminé, en fonction du siège de la discectomie, et de façon à rétablir les lordoses cervicale ou lombaire.

La face antérieure, plane, de dimensions variables, est percée en son centre d'un trou de fixation 53 taraudé au pas métrique iso, de profondeur et de diamètre variables, destiné à recevoir dans un premier temps le tournevis porte-cage et dans un second temps la vis de fixation de la plaque d'ostéosynthèse.

La face postérieure, de hauteur variable, comporte une dépression centrale arrondie 54, destinée à être positionnée en regard du ligament jaune.

Tous les bords et les coins des faces supérieure et inférieure de la cage inter-somatique 47 sont rayonnés.

### Exemple 10 : Clou centromédullaire

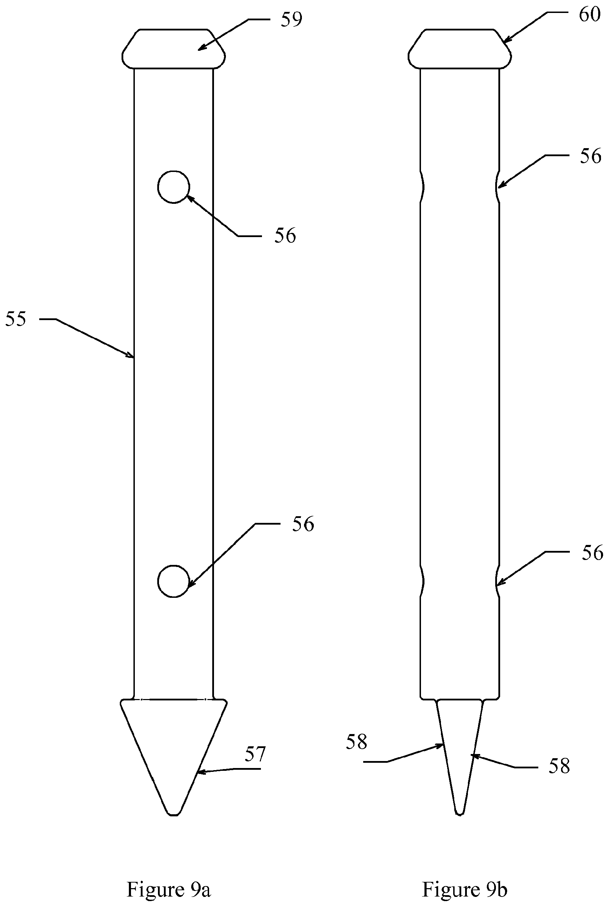

Un clou centromédullaire est obtenu selon le procédé décrit dans l'exemple 1. Les Figures 9a et 9b représentent respectivement une vue de face et une vue latérale du clou centromédullaire 55.

Le clou centromédullaire 55 se présente sous la forme d'un cylindre de diamètre et de longueur variables, comportant aux deux extrémités, deux ou plusieurs orifices 56 débouchants permettant de le claveter à l'aide de vis à appui bi-cortical.

L'extrémité inférieure 57 est en forme de harpon et comporte deux méplats 58 diamétralement opposés.

L'extrémité supérieure 59 est arrondie, aplatie au centre, afin de permettre l'impaction, et présente, sur tout son diamètre un rebord 60, au pourtour rayonné.

### Exemple 11 : Vis de contention membranaire

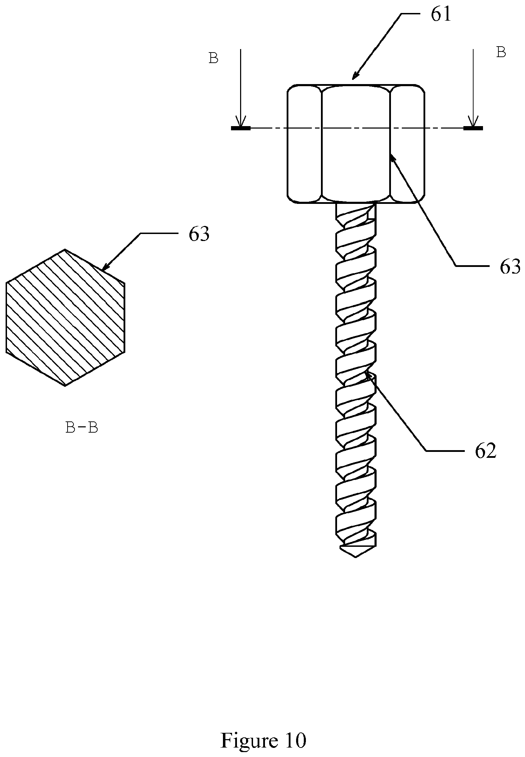

Une vis de contention membranaire est obtenue selon le procédé décrit dans l'exemple 1. La Figure 10 représente une vue de face de la vis de contention membranaire 61.

La vis de contention membranaire 61 se présente sous la forme de vis cylindrique de longueur et de diamètre variables, comportant un filetage 62 tel que décrit sur la Figure 6b, surmontée d'une tête hexagonale 63, de hauteur et de diamètre variables. Le pas de vis permet de s'opposer à l'arrachement lorsque la vis est mise en place dans une corticale ou un os alvéolaire déficient.

### Exemple 12 : Implant dentaire de substitution

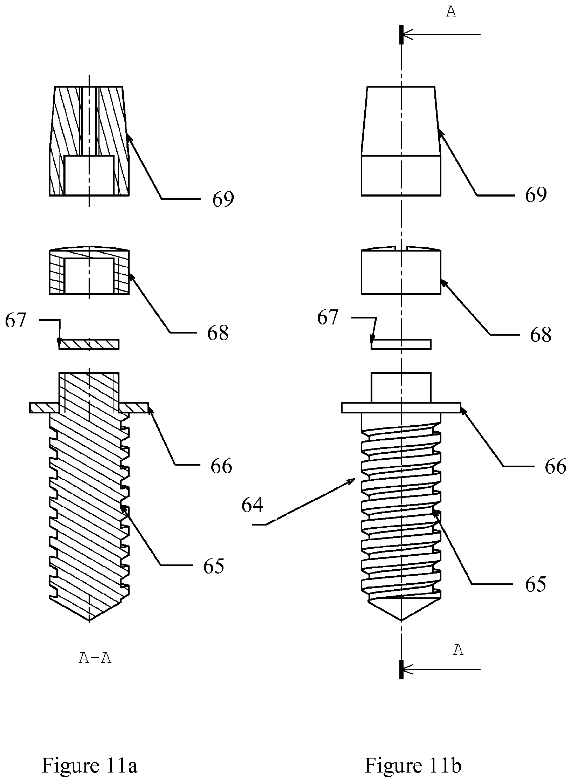

Un implant dentaire de substitution est obtenu selon le procédé décrit dans l'exemple 1. Les Figures 11a et 11b représentent respectivement une coupe sagittale et une vue éclatée de l'implant dentaire de substitution 64.

L'implant dentaire de substitution 64 comprend un corps 65 cylindrique de longueur et de diamètre variables, comportant un filetage s'opposant au dévissage tel que décrit sur la Figure 6b.

Le corps 65 est complété à son tiers supérieur, d'un anneau en feutre de Dacron® 66 destiné à favoriser une colonisation fibroblastique et réalisant une véritable sertissure gingivale sur tout son pourtour, délimitant ainsi une zone d'attachement gingival et une pseudo-gencive libre, s'opposant à la migration des fluides buccaux et des particules alimentaires.

Ce dispositif comporte également un disque de résilience 67, une coiffe 68 ainsi qu'un faux-moignon pré-prothétique 69, éléments en polymère biocompatible, destinés à recevoir la restauration prothétique.

### Exemple 13 : Résultats cliniques

On prépare, selon le procédé de l'exemple 1, 6 plaques épiphyso-diaphysaires en T avec 4 trous débouchants, dont la barre épiphysaire mesure 15 mm x 8 mm x 3 mm et la barre diaphysaire mesure 12 mm x 9 mm x 3 mm, et des vis de fixation selon l'exemple 7, de longueur 33 mm et de diamètre 3 mm, que l'on stérilise par rayonnements ionisants à 25 KGy sous double emballage. Ces plaques sont fixées sur la face externe de la région métaphyso-épiphysaire du tibia de 6 brebis selon un protocole chirurgical codifié.

Les animaux sont anesthésiés selon le protocole habituel : thiopental sodique (I V) 1g/animal approximativement, entretien isoflurane 1,7-6-1,8% et Kétamine avant les phases douloureuses. Après incision du plan cutané et dissection musculo-aponévrotique et périostique, la surface de l'os cortical est exposée.

A l'aide d'une plaque fantôme, munie des canons de perçage, et placée sur la zone d'insertion choisie, les orifices de fixation des vis du matériel d'ostéosynthèse et des ergots sont percés. Après retrait du matériel fantôme, les orifices de fixation sont taraudés de corticale à corticale, débouchants.

La plaque est positionnée et maintenue en place par des jauges de maintien, fixée par les vis en lieu et place des jauges de maintien.

Après hémostase, les plans profonds sont suturés avec des fils de suture résorbables, le plan cutané avec des fils non résorbables. Ces derniers sont enlevés après 10 jours de cicatrisation.

Des radios sont pratiquées à J+30 et J+60, les pièces anatomiques sont prélevées à J+60 sur trois brebis et à J+120 sur les trois autres brebis.

L'examen clinique des pièces anatomiques montre que les plaques et les vis sont parfaitement intégrées, recouvertes par le périoste.

Après réclinaison de celui-ci, les plaques et la tête des vis apparaissent opalescentes, imbibées de plasma, et adhérent à la corticale de l'os.

L'examen macroscopique montre une modification de la teinte des plaques d'ostéosynthèse, ainsi que la présence d'une suffusion d'un liquide jaune ambré, similaire au plasma, lors de la section de la plaque.

Cette observation laisse supposer que, durant le contact de la plaque avec la corticale et les fluides circulants, les feuillets bio-polymériques ont été imprégnés par ces derniers, via les pores interconnectés du matériau hybride semi-synthétique.

L'examen histomorphométrique des interfaces plaque-os cortical et vis-endoste montre une érosion lacunaire anfractueuse de la face interne de la plaque et des spires des vis, colonisée par des ostéoblastes, d'une apposition d'os métaplasique sous-périosté de même qu'on note un épaississement significatif de l'endoste.

L'interface vis-os cortical et vis-cavité médullaire montre les mêmes images, à savoir une érosion lacunaire en surface le long des spires des vis et sur toute leur longueur, avec apposition d'os jeune néoformé, ce qui laisse supposer que les cellules souches mésenchymateuses de la moelle osseuse en sont à l'origine, avec l'induction d'une activité ostéo-promotrice.

Toutes ces observations témoignent d'une activité biologique interactive entre le matériau hybride semi-synthétique selon l'invention et l'os receveur.

En effet, il a été démontré que la présence initiale de cellules géantes ostéoclastiques, trouvées à proximité des vis dans la cavité médullaire, formées par fusion, allant jusqu'à 100 µm, et dérivant des mêmes cellules précurseurs que les monocytes, montre qu'elles sont responsables de la formation des lacunes en formes d'anfractuosités à la surface des vis. On peut comparer ces anfractuosités aux lacunes de Howship, caractéristiques de l'activité ostéoclastique, témoin du remodelage osseux. En d'autres termes, ces observations rendent compte des propriétés ostéomimétiques de la plaque et des vis d'ostéosynthèse ou des implants, selon l'invention.

Toutes ces caractéristiques du matériau semi-synthétique hybride, objet de l'invention, permettent de proposer une solution alternative aux problèmes liés à la présence et/ou à l'ablation du matériel d'ostéosynthèse métallique, tels que relargage toxique, métallose des tissus périprothétiques, effets systémiques, fragilisation de l'os, etc....

Cela justifie le concept d'un matériel d'ostéosynthèse pérenne, ne nécessitant plus l'ablation du matériel orthopédique, pour quelque raison que ce soit.

## Revendications

1. Matériau d'ostéosynthèse hybride semi-synthétique qui est la couche aragonitique nacrée de mollusques bivalves choisis dans le groupe comprenant Pinctada Maxima, Pinctada Margaritifera, Tridacnae Maxima, Tridacnae Gigas et autres Pinctadines, ledit matériau comprenant une fraction inorganique et une fraction organique , réticulée et présentant un pH de 7 à 7,4.

2. Procédé de fabrication d'un matériau d'ostéosynthèse hybride semi-synthétique tel que défini dans la revendication 1 à partir d'un biomatériau hybride naturel qui comprend une fraction inorganique et une fraction organique,
**caractérisé en ce que** le biomatériau hybride naturel est la couche aragonitique nacrée de mollusques bivalves choisis dans le groupe comprenant Pinctada Maxima, Pinctada Margaritifera, Tridacnae Maxima, Tridacnae Gigas et autres Pinctadines, et
**en ce que** ledit procédé comprend une étape de modification du pH et une étape de réticulation de la fraction organique dudit biomatériau hybride naturel.

3. Procédé de fabrication d'un dispositif d'ostéosynthèse ou implant en matériau hybride semi-synthétique tel que défini dans la revendication 1, comprenant les étapes de:
a) Sélection de valves, ayant auparavant subi l'exposition de la couche aragonitique nacrée, des mollusques choisis dans le groupe comprenant Pinctada Maxima, Pinctada Margaritifera, Tridacnae Maxima, Tridacnae Gigas et autres pinctadines,
b) Découpe de préformes et fabrication du matériel d'ostéosynthèse, éventuellement après modélisation numérique,
c) Modification de pH et réticulation, et
d) Modification de l'état de surface.

4. Procédé selon la revendication 2 ou 3, **caractérisé par le fait que** l'étape de modification du pH est réalisée par immersion dans un bain d'eau du réseau microbiologiquement maîtrisée, portée à ébullition.

5. Procédé selon l'une quelconque des revendications 2 à 4, **caractérisé en ce que** l'étape de réticulation est réalisée à l'aide d'un agent réticulant tel que la riboflavine, avec exposition actinique aux UVA, ou la vitamine C, un polyol tel que le mannitol, et/ou des agents physiques tels que les rayonnements ionisants, de préférence la riboflavine avec exposition aux UVA.

6. Procédé selon l'une quelconque des revendications 3 à 5, dans lequel l'étape d) de modification de l'état de surface comprend des opérations de sablage, nettoyage par ultrasons, cryogénie et application de nanoparticules mécano-structurées dudit biomatériau hybride naturel.

7. Procédé selon l'une quelconque des revendications 3 à 6, comprenant en outre une étape e) d'imprégnation par des liquides biologiques et/ou des compositions contenant des substances pharmaceutiquement actives, du dispositif obtenu après l'étape d).

8. Dispositif d'ostéosynthèse ou implant en matériau hybride semi-synthétique tel que défini dans la revendication 1, ou obtenu selon le procédé de l'une quelconque des revendications 3 à 7, qui est choisi parmi des dispositifs d'ostéosynthèse tels que plaque d'ostéosynthèse droite, plaque d'ostéosynthèse épiphyso-diaphysaire, plaque d'ostéosynthèse pour fracture malléolaires, plaque ruptive d'ostéosynthèse épiphyso-diaphysaire ; vis d'ostéosynthèse ; vis de contention membranaire ; coins d'ostéotomie ; diabolos ; cales et cages inter-somatiques ; clous centromédullaires ; têtes humérales et fémorales ; cavités glénoïdes ; plateaux tibiaux ; condyles fémoraux ; corps vertébraux ; hémi-maxillaires ; os de la chaîne des osselets ; ancres chirurgicales pour la réinsertion ligamentaire et/ou tendineuse ; gouttières gainantes pour la réduction ostéosynthésée de fractures comminutives à petits fragments et implants dentaires.

9. Dispositif selon la revendication 8, **caractérisé en ce qu'**il comporte au moins un moyen de contention qui, lors de la pose dudit dispositif s'oppose à son déplacement, ledit moyen de contention étant choisi parmi un ergot, une encoche, un harpon claveté et aplati, un filetage s'opposant au dévissage.

10. Dispositif selon la revendication 8 ou 9, qui comprend en outre un moyen d'ajustement qui, lors de la pose dudit dispositif, permet sa coaptation.

11. Dispositif selon l'une quelconque des revendications 8 à 10, qui est une plaque ruptive d'ostéosynthèse épiphyso-diaphysaire et qui comprend une encoche de rupture.

## Patentansprüche

1. Semi-synthetisches Hybrid-Osteosynthesematerial, welches die perlmuttartige Aragonitschicht von Muscheln ist, gewählt aus der Gruppe, umfassend Pinctada Maxima, Pinctada Margaritifera, Tridacnae Maxima, Tridacnae Gigas und andere Pinctadine, wobei das Material einen inorganischen Anteil und einen vernetzten organischen Anteil mit einem pH-Wert von 7 bis 7,4 aufweist.

2. Herstellungsverfahren für ein semi-synthetisches Hybrid-Osteosynthesematerial wie in Anspruch 1 definiert, basierend auf einem natürlichen Hybrid-Biomaterial, welches einen inorganischen Anteil und einen organischen Anteil aufweist,
**dadurch gekennzeichnet, dass** das natürliche Hybrid-Biomaterial die perlmuttartige Aragonitschicht von Muscheln ist, gewählt aus der Gruppe, umfassend Pinctada Maxima, Pinctada Margaritifera, Tridacnae Maxima, Tridacnae Gigas und andere Pinctadine, und
dadurch, dass das Verfahren einen Schritt der Modifikation des pH-Wertes und einen Schritt der Vernetzung des organischen Anteils des natürlichen Hybrid-Biomaterials umfasst.

3. Herstellungsverfahren für eine Osteosynthesevorrichtung oder -implantat aus semisynthetischem Hybridmaterial wie in Anspruch 1 definiert, welches die folgenden Schritte umfasst:
a) Selektion von Schalen, welche zuvor der Exposition der perlmuttartigen Aragonitschicht von Muscheln, gewählt aus der Gruppe, umfassend Pinctada Maxima, Pinctada Margaritifera, Tridacnae Maxima, Tridacnae Gigas und andere Pinctadine unterzogen wurden,
b) Ausschneiden von Vorformen und Herstellung des Osteosynthesematerials, eventuell nach numerischer Modellierung,
c) Modifikation des pH-Wertes und Vernetzung, und
d) Modifikation des Oberflächenzustands.

4. Verfahren nach Anspruch 2 oder 3, **dadurch gekennzeichnet, dass** der Schritt der Modifikation des pH-Wertes durch Eintauchen in ein zum Kochen gebrachtes Wasserbad des mikrobiologisch beherrschten Netzes erfolgt.

5. Verfahren nach einem der Ansprüche 2 bis 4, **dadurch gekennzeichnet, dass** der Schritt der Vernetzung mittels eines Vernetzungsmittels erfolgt, wie beispielsweise Riboflavin, unter aktinischer UVA-Exposition, oder Vitamin C, ein Polyol, wie beispielsweise Mannitol, und/oder physikalische Mittel wie ionisierende Strahlung, bevorzugt Riboflavin mit UVA-Exposition.

6. Verfahren nach einem der Ansprüche 3 bis 5, wobei der Schritt d) der Modifizierung des Oberflächenzustands Sandstrahlvorgänge, Reinigung durch Ultraschall, Kryotechnik und die Anwendung von mechanisch strukturierten Nanopartikeln des natürlichen Hybrid-Biomaterials umfasst.

7. Verfahren nach einem der Ansprüche 3 bis 6, welches ferner einen Schritt e) der Imprägnierung durch biologische Flüssigkeiten und/oder Zusammensetzungen mit pharmazeutischen Wirkstoffen der nach Schritt d) erhaltenen Vorrichtung umfasst.

8. Osteosynthesevorrichtung oder -implantat aus semisynthetischem Hybridmaterial wie in Anspruch 1 definiert, oder durch das Verfahren nach einem der Ansprüche 3 bis 7 erhalten, welches gewählt ist aus Osteosynthesevorrichtungen wie beispielsweise gerade Osteosyntheseplatte, epiphyso-diaphysäre Osteosyntheseplatte, Osteosyntheseplatte für eine Knöchelfraktur, unterbrochene epiphyso-diaphysäre Osteosyntheseplatte; Osteosyntheseschraube; Schraube zur Membranhalterung, Osteotomie-Ecken, Diabolos, Zwischenkörperkeile und -käfige, Marknägel, Oberarm- und Hüftköpfe, Gelenkpfannen, Tibiaplateaus, Femurkondylen, Wirbelkörper, Halb-Kiefer, Knochen der Gehörknöchelchenkette, chirurgische Verankerungen für die Reinsertion von Bändern und/oder Sehnen, umhüllende Rinnen für die osteosynthetische Reduktion von Splitterfrakturen mit kleinen Fragmenten und Dentalimplantate.

9. Vorrichtung nach Anspruch 8, **dadurch gekennzeichnet, dass** sie wenigstens ein Haltemittel umfasst, welches bei der Anbringung der Vorrichtung ihrer Verlagerung entgegenwirkt, wobei das Haltemittel gewählt ist aus einem Sporn, einer Vertiefung, einem verkeilten und abgeflachten Haken, einem das Lösen verhindernden Gewinde.

10. Vorrichtung nach Anspruch 8 oder 9, welche ferner ein Regulierungsmittel umfasst, welches bei der Anbringung der Vorrichtung ihre Verbindung ermöglicht.

11. Vorrichtung nach einem der Ansprüche 8 bis 10, welche eine unterbrochene epiphyso-diaphysäre Osteosyntheseplatte ist und eine Sollbruchkerbe umfasst.

## Claims

1. A semi-synthetic hybrid osteosynthesis material, which is the nacreous aragonitic layer of bivalve mollusks selected from the group consisting of *Pinctada maxima, Pinctada margaritifera, Tridacnae maxima, Tridacnae gigas* and other Pinctada species, said material comprising an inorganic fraction and a cross-linked organic fraction and having a pH from 7 to 7.4.

2. A method for producing a semi-synthetic hybrid osteosynthesis material as definied in claim 1 from a natural hybrid material which comprises an inorganic fraction and an organic fraction,
**characterized in that** the natural hybrid biomaterial is the nacreous aragonitic layer of bivalve mollusks selected from the group consisting of *Pinctada maxima, Pinctada margaritifera, Tridacnae maxima, Tridacnae gigas* and other Pinctada species, and
**in that** said method comprises a step of modifying the pH and a step of cross-linking the organic fraction of said natural hybrid biomaterial.

3. A method for producing an osteosynthesis device or implant made of the semi-synthetic hybrid material as defined in claim 1, comprising the steps of:
a) selecting valves, having previously undergone exposure of the nacreous aragonitic layer, of the mollusks selected from the group consisting of *Pinctada maxima, Pinctada margaritifera, Tridacnae maxima, Tridacnae gigas* and other Pinctada species,
b) cutting out pre-forms and producing the osteosynthesis material, if appropriate after digital modeling,
c) modifying the pH and cross-linking, and
d) modifying the surface state.

4. The method according to claim 2 or 3, wherein the step of modifying the pH is performed by immersion in a bath of microbiologically controlled mains water, brought to boiling point.

5. The method according to any one of claims 2 to 4, wherein the cross-linking step is performed with the aid of a cross-linking agent such as riboflavin, with actinic exposure to UVA, or vitamin C, a polyol such as mannitol, and/or physical agents such as ionizing radiation, preferably riboflavin with exposure to UVA.

6. The method according to any one of claims 3 to 5, wherein the step d) of modifying the surface state comprises operations of sandblasting, cleaning by ultrasound, cryogenics and application of mechanically structured nanoparticles of said natural hybrid biomaterial.

7. The method according to any one of claims 3 to 6, further comprising a step e), wherein the device obtained after step d) is impregnated by biological liquids and/or compositions containing pharmaceutically active substances.

8. An osteosynthesis device or implant made of the semi-synthetic hybrid material as defined in claim 1 , or obtained by the method according to any one of claims 3 to 7, which is chosen from osteosynthesis devices such as a straight osteosynthesis plate, an epiphyseal-diaphyseal osteosynthesis plate, an osteosynthesis plate for malleolar fractures, an epiphyseal-diaphyseal osteosynthesis break plate; osteosynthesis screws; membrane retention screws; osteotomy wedges; diabolos; intersomatic wedges and cages; intramedullary nails; humeral and femoral heads; glenoid cavities; tibial plateaus; femoral condyles; vertebral bodies; semi-maxillaries; bones of the ossicular chain; surgical anchors for ligament and/or tendon reinsertion; splints for osteosynthesized reduction of small-fragment comminuted fractures, and dental implants.

9. The device according to claim 8, **characterized in that** it comprises at least one retention means which, during the positioning of said device, opposes the displacement thereof, said retention means being chosen from among a pin, a notch, a keyed and flattened harpoon, a thread against unscrewing.

10. The device according to claim 8 or 9, further comprising an adjustment means which, during the positioning of said device, permits coaptation thereof.

11. The device according to any one of claims 8 to 10, which is an epiphyseal-diaphyseal osteosynthesis break plate and which comprises a break notch.
